Europäisches Patentamt

European Patent Office

Office européen des brevets

(19)

(11) Publication number: **0 291 569 B1**

## EUROPEAN PATENT SPECIFICATION

(12)

(45) Date of publication of patent specification: **01.06.94**   (51) Int. Cl.⁵: **A61N  5/06**

(21) Application number: **87114271.7**

(22) Date of filing: **30.09.87**

The file contains technical information submitted after the application was filed and not included in this specification

(54) **Method and apparatus for treatment by radiation matched with the human body's frequency spectrum.**

(30) Priority: **20.05.87 CN 87103603**
**20.05.87 CN 87208158**

(43) Date of publication of application:
**23.11.88 Bulletin  88/47**

(45) Publication of the grant of the patent:
**01.06.94 Bulletin  94/22**

(84) Designated Contracting States:
**DE FR GB IT NL**

(56) References cited:
DD-A- 221 374          DE-A- 2 340 928
DE-A- 3 321 825        US-A- 1 922 079
US-A- 3 822 693        US-A- 4 023 566
US-A- 4 246 905        US-A- 4 622 952
US-A- 4 622 953

**SOVIET INVENTIONS ILLUSTRATED; section P,O, week 87/19, 24 June 1987DERWENT PUB-LICATIONS LTD., London, p 34**

**SOVIET INVENTIONS ILLUSTRATED section P,O, week C51,4 February 1981DERWENT PUBLICATIONS LTD., London, p 33**

(73) Proprietor: **Zhou, Lin**
**Apt. 232**
**Huashan Lane 2**
**Wucheng Road**
**Kunming Yunnan(CN)**

Proprietor: **Zhang, Xue-shan**
**Apt. 232**
**Huashan Lane 2**
**Wucheng Road**
**Kunming Yunnan(CN)**

(72) Inventor: **Zhou, Lin**
**Apt. 232**
**Huashan Lane 2**
**Wucheng Road**
**Kunming Yunnan(CN)**
Inventor: **Zhang, Xue-shan**
**Apt. 232**
**Huashan Lane 2**
**Wucheng Road**
**Kunming Yunnan(CN)**

(74) Representative: **Liesegang, Roland, Dr.-Ing. et al**
**FORRESTER & BOEHMERT**
**Franz-Joseph-Strasse 38**
**D-80801 München (DE)**

## Description

This invention belongs to the realm of medical apparatus and its manufacture. Its apparatus has special curative effects to cure the diseases of chilblaines, frostbite,burns and scalds, gynecology such as dysmenorrhoea, chronic ulcer of the skin, arthritis, periarthritis of the shoulder, coutusion of soft tissure bronchitis, asthma, functional disorder in stomach and intestines,hypertension,healing of wound and so on.

Presently, the popular physiotherapy equipment are mainly those of electrotherapy, ultrasonic wave, infrared rays,ultraviolet rays, microwave, laser, warm-heat treatment and so on. They are used to cure many sorts of diseases. When they are used to cure human body's diseases, the physiotherapy equipment of ultrasonic wave emits ultrasonic wave with frequency of 20,000 Hz or above to act on the human body; the physiotherapy equipment of infrared rays emits infrared rays with wavelength of 780 - 30,000 millimicrons to irradiate the human body; the physiotherapy equipment of ultraviolet rays produces ultraviolet rays with wavelength of 180 - 380 millimicrons to irradiate the human body; the physiotherapy equipment of microwave emits electromagnetic wave of ultrahigh frequency with wavelength of 1 - 100 millimeters to act on the human body; and the physiotherapy equipments of electrotherapy, laser and warm-heat treatment use the equipment of electric conductance, irradiating of monochromatic stimulated radiant light and thermal conductance respectively to cure diseases. In certain historic period, the said physiotherapy equipments have all made many beneficial contributions to conquer sufferings from some diseases and pains for humanity to varying degrees. But with the development of society, people's reuirements and desires are also developed, so that, shortcomings of the said physiotherapy equipments are gradually known and experienced by people. These shortcomings are mainly as follows:

(1) The curative effects are much limited. The range of diseases to be cured by each physiotherapy equipment singly is limited and therefore some common diseases ( such as chilblaibs, frostbite, rhinitis, cold ,etc., ) can not also get fine cures rapidly and effectively.

(2) Higher factory costs, and they are all inconvenient to be manufactured, maintained and operated, and on the other hand, there are often side effects from using them.

(3) It is difficult to suit the use for the domestic medical health care and in the small-scale medical units .

And those installations, which are being used presently for domestic medical health care or lighting,have relatively good functions of medical health protection and general lighting,and advantages of low costs,being convenient for the use and maintenance and so on as well,are far from meeting the requirements of people's daily life and medical health protection both in kinds or their effects.

For example these prior arts such as ones which application numbers are respectively DT2846221, WO8302233, DE3301802 and CN85100538, although they offer ultraviolet rays, carrying lights and near-infrared rays to treat human diseases,but owing to their limitations in the range and effect of treatments for human diseases by these rays and the problems such as daily lightings etc are less considered,they can not still satisfy the requirements of people's daily lighting and medical health care at the same time.

The purpose of this invention is to offer a kind of radiation, or "effect-field" treatment apparatus matched with the human body's frequency spectrum and its production method. When it is used to cure diseases of human body, the apparatus can emit electromagnetic radiation or "effect-field" which is the same or similar to the natural frequency, i.e., the matching effect-field of human body's frequency spectrum, by means of better imitation of the healthy human body's natural frequencies of this frequency range in a wider frequency range. When it acts on the patient's body, the effect-field can effectively solve those problems of poor clinical curative effects for some common diseases. This apparatus shall have so multi-functions according to the requirement such as to cure effectively or auxiliarily many sorts of diseases in the departments of internal medicine, surgical department, paediatrics, gynaecology and obstetrics, dermatology and so on, and can be small-sized also. The production method of this apparatus shall be easily mastered by ordinary engineers and technicians of this specific field with better engineering stability and it has advantages of less investment and early effectiveness.

The another purpose of this invention is to offer a kind of double-function lamp,which has functions of daily medical health protection and living lighting at the same time,so it has curative effects for many sorts of diseases and also has notable curative effects partly or completely on three ranges of treatments for curing the diseases of blood circulation system,the sorts of diseases of the surgical hurts and skins as well as arthritis,and the diseases of autonomic neurosis, it is also a more simple and reasonable designed model which sufficiently considers the requirements of daily lighting.

The invention is defined in the appended claims.

The imitating generator of frequency spectrum imitating the frequency spectrum of the human's body consists of the imitating or "genetic" layer of frequency spectrum, heating layer and matrix. The imitating or "genetic" layer of frequency spectrum, which is made up by using fourteen sorts of sufficiently mixed materials mentioned in the above technical formulations and then pulverizing these selected materials by ball mill into powder with grain size of about 50 meshes and finally compounding them to a solution with proper amount of silicasol added, is coated on the exterior surface of the heating layer. The heating layer is compounded into solution by the materials of $SnCl_4 : SbCl_3 : FeCl_3$ , sprayed onto the matrix, hydrolyzed and oxidized under high temperature and formed an electrical conductive layer of polycrystalline semi-conductor (metalic oxides) with it electrical power controlled by putting some impurities into the materials. Basically, the matrix may be made up by burning ordinary heat-resistant materials.

When this invention is used clinically, some medicines may be also used at the same time for getting better curative effects. For example, to cure chilblains and frostbite, the tinctures No.1 or 2 prescription may be used for auxiliarily treatment. Here, the No. 1 prescription is : 10 grammes of camphor and alcohol 1000 ml with concentration of 95 % compounding a tinct; and the No.2 prescription is : 10 grammes of white pepper and alcohol 1000 millilitres with concentration of 95%, these two kinds of tinctures will be coated on the non-festered pats homogeneously. When this invention is used to cure the contusion and sprain of soft tissue so as to achieve effects of invigorating the circulation of blood, stimulating the menstrual flow, expelling rheumarthritis or stopping pain and rheumarthritis etc., No.3 or No.4 prescriptions may be used by coating them on the festered parts. Here the No. 3 prescription is : a few safflower and some alcohol with concentration of 95% compounding to a tincture; the No.4 prescription is to compound a tincture of the root of fangji (Stephania tetrandra) or the rhizome of chuanxing (Ligusticum wallichii), and the compound Baiyao or anodyne plaster of wound and rheumatism for external pasting can be also used.If these medicines are to be used, all of them should firstly be applied and coated or pasted to the affected parts before receiving treatment of this apparatus.

For the purpose to develop curative functions of this inventive apparatus, some music curative devices are added in this apparatus so that the patient may receive music treatment while receiving the matching treatment of frequency spectrum which is helpful to recover the systematic function of brain nerve, postpone the decrepitude of brain, eliminating the feeling of strain and weariness, improving the state of spirit and body, and curing some diseases in body and mind as well. such a multifunctional apparatus can both save time and let patients receive the treatments for many sorts of diseases in a happy and comfortable curative environment.

Compared with the prior arts, this invention has obvious advantages for its effective rate, quick effectiveness and easy to handle,and the more important characteristic is that the treatments for some common diseases have notable curative effects which is impossible by using the routine technical installations and methods. For example, their treatments to the chilblains and frostbite have been stagnated for a long time, each of the treating methods has no good curative effects to treat them until now and there are at least thirty million patients suffering from them waiting effective treatments, but this invention can solve this problem simply and effectively, it can offer effects to stop itch and pain in the first day of receiving the treatment, the wounded and festered surfaces are obviously dried, the cure can be achieved only in 2 to 7 times of treatments with stable efficiency about 98% . On the other hand, according to the function principle of this invention, different structures of human bodys and treating requirements of different diseases, after the imitating frequency spectrum, the area, surface color and dosages of its generator have been adjusted technically, this invention has preliminarily been discovered to have so large a suitable range as to offer better curative effects and auxiliary curative effects to many diseases of micro-circulation obstruction and inflammation or infection, see Table 1 for the partial clinical cases of illnesses. The coming out of this invention can offer a kind of treating apparatus and method to cure and prevent diseases which are simple, effective, low cost and no side effects, and at the same time it can even save enormous resources of medicines. The curative apparatus offered by this invention can offer an electromagnetic wave effect-field matching with the natural frequency of healthy human body in a certain range of frequency.

Owing to the fact that the above main technical solutions are adopted, this lampe can principally have the functions both for illumination as well as prevention and treatment of diseases with one lamp only,and also because the radiators are made of selected proportions of element materials in three groups, and the radiators can simulate to emit effect fields by means of imitating human body's frequency spectrum, which can concurrently cure many sorts of diseases in the department of internal medicine, surgical department, gynaecology and obstetrics,dermatology, paediatrics and so on, and can also,according to the selected different model of supports or different radiators,offer the functions having notably curative effects to cure respectively or simultaneously the diseases of blood circulation system ( with Group I ), the sorts of diseases of the surgical hurt and skins as well as the arthritis ( with Group II ), and the autonomic neurosis ( with Group III ), and on the other hand it has the advantages of simple design, proper constructions and being convenient to be made and used, therefore, this double-function lamp is more suitable to be used

EP 0 291 569 B1

domestically.

For having gotten those advantages mentioned above, the purposes of this invention have been basically accomplished.

The following contents are the brief descriptions of the attached figures and tables in this specification :

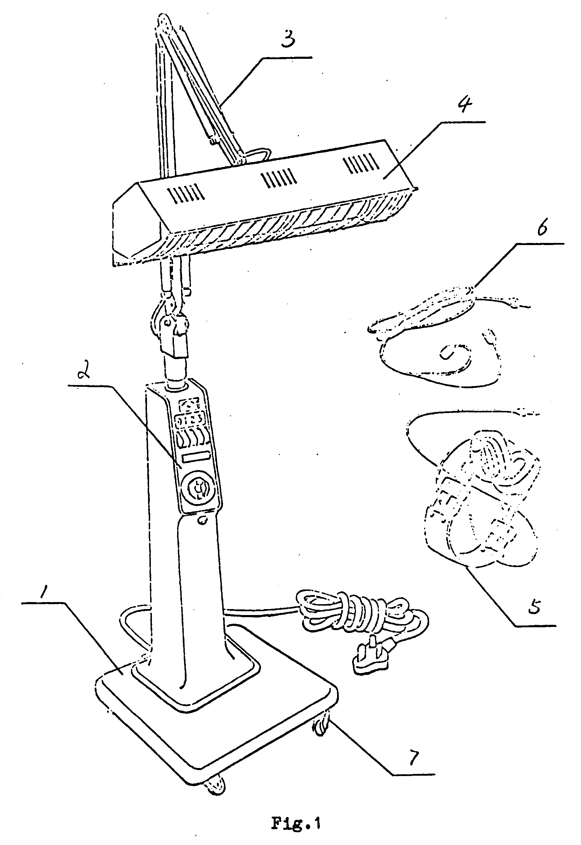

Fig.1. The embodiment sketch map of this matching effect-field treatment apparatus of human body's frequency spectrum.

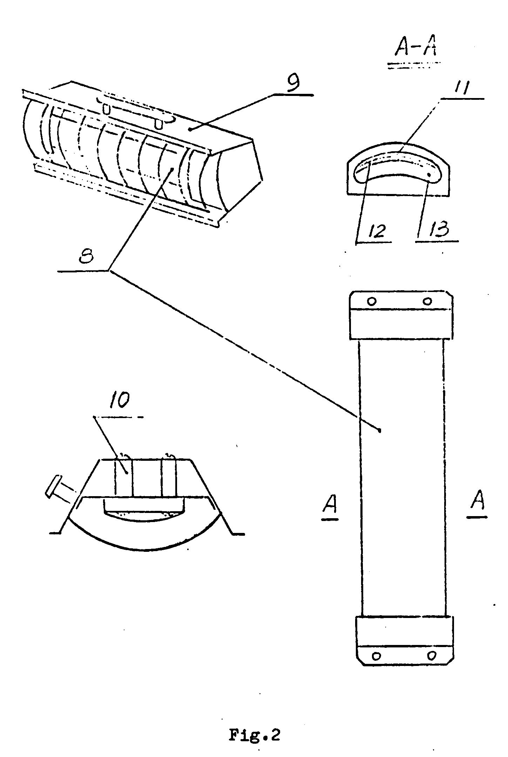

Fig.2. The structural sketch map of matching effect-field generator of human body's frequency spectrum.

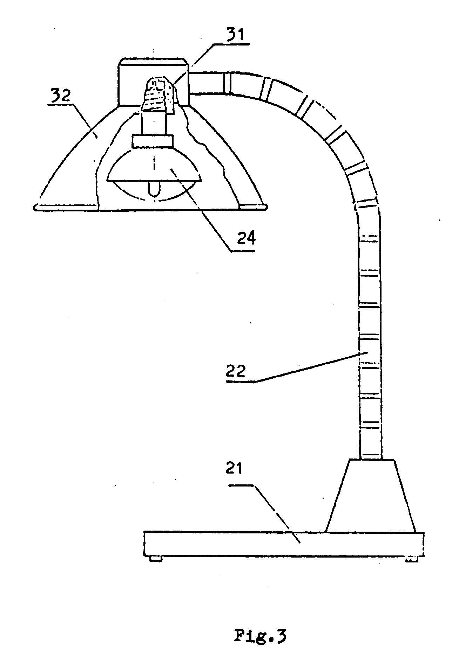

Fig.3.sketch map of the structure of a unilateral and single-group treatment-lighting lamp.

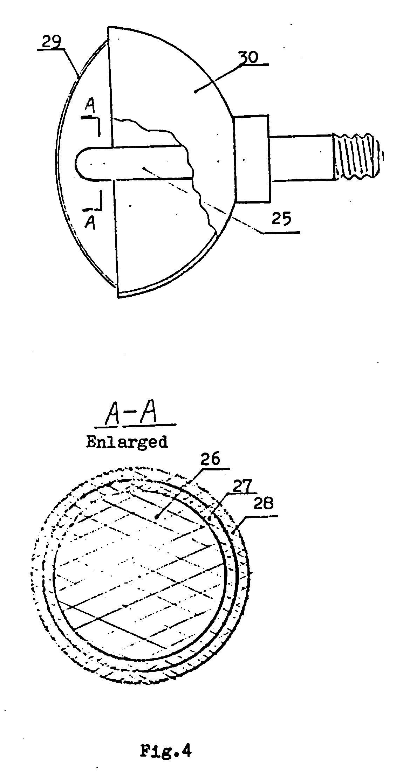

Fig.4. sketch map of the structure of a treatment lamp-holder for a unilateral and single-group lamp.

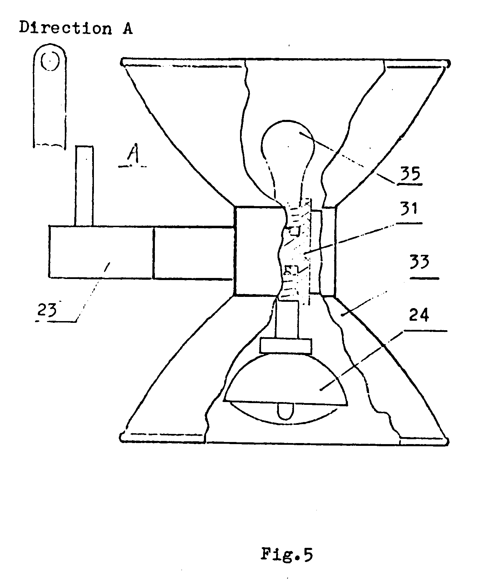

Fig.5. sketch map of the structure of a treatment-lighting lamp-holder for a bilateral and single-group lamp.

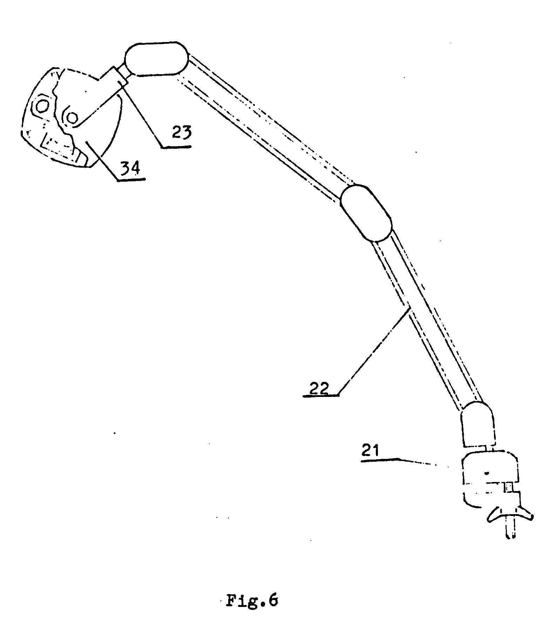

Fig.6. sketch map of the structure of a polyhedral and multi-group treatment-lighting lamp.

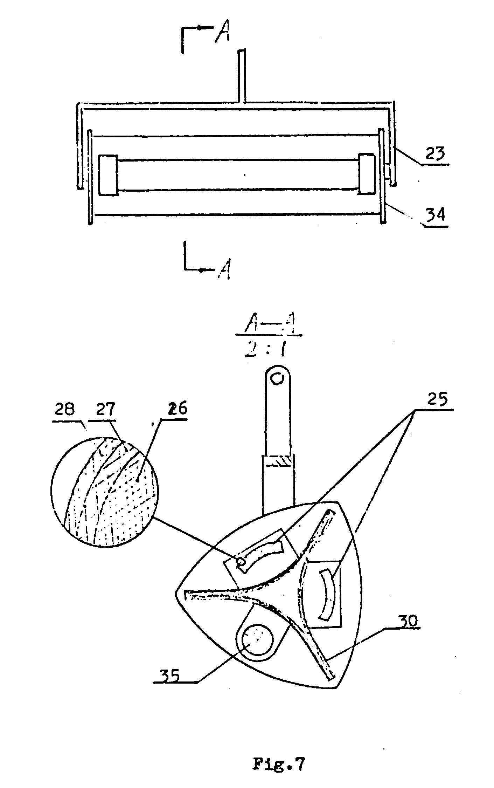

Fig.7. sketch map of the structure of a treatment-lighting lamp-holder for a trihedral and two-group treatment-lighting lamp.

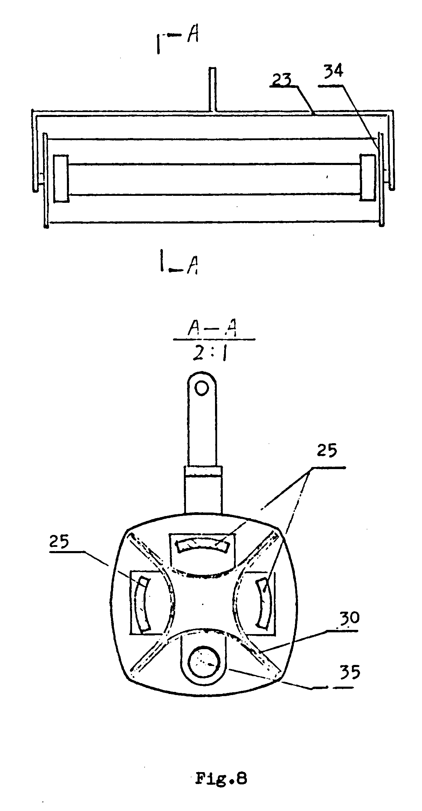

Fig.8.sketch map of the structure of a treatment-lighting lamp-holder for a tetrahedral and three-group treatment-lighing lamp.

Tab.1. Some clinical cases of illnesses treated by this matching effect-field treatment apparatus of human body's frequency spectrum.

The symbols in the above figures mean respectively as follows :

(1) Machine base;

(2) Controller of instruments;

(3) Supporting arm;

(4) Effect-field generator and its accessories;

(5) Earphone;

(6) Stimulant electrode;

(7) Universal wheel;

(8) Effect-field generator;

(9) Reflex protective cover;

(10) Fixed strut;

(11) Imitating genetic layer of human body's frequency spectrum;

(12) Transducing layer;

(13) Matrix;

(21) pedestal;

(22) Lamp rod;

(23) Support;

(24) Pingle-group treatment lamp-holder;

(25) Radiator;

(26) Matrix;

(27) Transducing layer;

(28) Imitating genetic layer of human body's frequency spectrum;

(29) Protective network;

(30) Reflex cover;

(31) Lamp base;

(32) Lamp cover;

(33) Bilateral lamp cover;

(34) Rolling support buckle;

(35) Bulb or tube.

One embodiment of this invention is as follows :

The whole apparatus offered in this embodiment consists of the machine base (1), controller (2) of instruments, supporting arm (3), effect-field generator and its accessories (4), earphone (5), stimulant electrode (6) and universal wheels (7). The machine base is comprised of the two parts, the flat parallel base-plate and the base-rod. The base-rod is fixed in the centre of the base-plate. The universal wheel (7) is fixed on the bottom of the base-plate. The controller (2) of instruments, in which various electric elements and components are installed, is fixed in the centre of base-rod. One end of the supporting arm (3) is mechanically connected with the said base-rod in the top of the base-rod, while the other end of the said supporting arm (3) is connected with the effect-field generator and its accessories. The earphone (5) and stimulant electrode (6) are connected with the relative electrical appliances in the controller (2) of instruments by means of electrical connectors.

5

The effect-field generator (8) is installed in the effect-field generator and its accessories (4). The matrix (13) is set in the effect-field generator (8), and the transducing layer (12) is metallized on the matrix (13) while the imitating genetic layer (11) of human body's frequency spectrum is coated on the transducing layer (12). The transducing layer (12) is circuitally connected with the transducing control circuits. The matrix (13) is set in the centre of the reflex protective cover (9). The effect-field generator (8) is fixed on the supporting arm (3) by the fixed strut (10) of the generator.

The imitating genetic layer (11) of human body's frequency spectrum is so prepared as follows : The said 14 sorts of element constituents which make up the imitating genetic layer of human body's frequency spectrum are proportional prepared, mixed and then pulverized into powder with grain size of 50 meshes by ball geinder.Then the pulverized materials are calcines for 3 hours under high temperature of 1140 ° C, taken out and again pulverized into particles also with the 50 meshes for the purpose of doing best to get any one of the particles contains the said 14 sorts of element constituents. After that, certain amounts of cilicasol binder is put into these particles and homogeneously stirred so as to get a kind of liquid and this liquid is vacuum coated onto the external surface of the transducing layer (12), and then put them into the bake-out furnace which already preheated to about 150 ° C and solidify for 1 hour to finish the preparation.

The transducing layer (12) of semi-conductor is so prepared as follows : The materials of tin tetrachloride, stibium trichloride and iron trichloride are so mixed with the proportion of tin tetrachloride : stibium trichloride : iron trichlorid = 105 : 0.8 : 1 as to get a kind of solution and this solution is evenly metallized on the clean matrix (13) of effect-field generator. Then put them under temperature of 800 ° C and hydrolyze to sosoloid, that is, to generate an electrical conductive layer of metallic oxide, which is also called the electrical transducing layer of hole-semiconductor and electron-semiconductor, on the surface of matrix (13) , and after that, coat silver films on both end of the electrodes of the matrix (13) so as to reduce the contact resistance between transducing layer (12) and power supply. Being electrified, the transducing layer can then transfer energy to supply the imitating genetic layer (11) of human body's frequency spectrum. Power of the transducing layer is determined by its surface resistance. (It is 300W in this embodiment ).

The matrix (13) of effect-field generator is so prepared as follows : It adopts common heat-resistant insulating materials as the row materials and designed as well as prepared or formed according to the requirements.

The fixed strut (10) of the effect-field generator (8) is so prepared as follows : Use materials such as polytetrafluoroethylene etc and form post shape It should satisfy the requirements of insulation and heat insulation as well as having internal threads.

The reflex protective cover (9) is so prepared as follows : To punch aluminium sheets with thickness of 0.8 - 1 millimeter respectively into the reflex protective cover and the front window-protective plate according to the designed size ( it is 300 millimeters long, 120 millimeters wide and 50 millimeters thick in this embodiment ). Then they are surface anodic oxidated and polished. The effect-field generator is fixed with the reflex protective cover by the heat-insulating and insulating fixed strut (10) and the wires of power supply passing through the cover are of the high temperature type .

The curative music instrument is completed with the stereosonic source of wideband and two-channels and the earphone (5), and it has music-controlled acupoint-stimulant electrodes being able to lead the stimulating currents controlled by music into acupoint of human body,thus , to offer required music-treatments according to different patients with different melody of music tape.

For the convenience of understanding and using this apparatus, the following examples are given to illustrate the methods of curing some diseases.

(1) Chilblains and frostbite: If there are broken or festered surfaces, the wound must be cleaned firstly and made the surface to be as dry as possible. And then use the relative curative apparatus of this invention for the treatment. Time for the treatment is 15 to 60 minutes and the distance between the generator of frequency spectrum and the affected part is 50 to 500 mm. The acting area of the generator of frequency spectrum must be able to cover the affected area. The optimum treating dosage is selected on the basis of sensible intensity for the patient, and the treating voltage is set on 80 to 200 V. Ordinary patients may be cured after 2 to 7 times of treatment. And for those patients hasing wounds with itch and pain can get the curative effects of stopping itch and pain as well as making the surface of wound obviously dry on the first day.

(2) Chronic ulcers caused by the disease such as vasculitis and varix on the lower limbs : If it is of one which has secretions, the surface of wound must be cleaned firstly and keep dry before using the relative apparatus of this invention for the treatment. The generator of frequency spectrum will be placed in the concave-arc form on the basis of the form of affected limbs and the acting area of the generator of frequency spectrum must cover the affected limb with a distance between the generator of frequency spectrum and the limbs about 50 - 500 mm. The optimum treating voltage is selected according to the fact of feeling warm and comfortable for the patients and in genearl conditions it is about 80 - 150 V. Ordinary patients may get obvious improvement after 3 - 10 times of treatment, but it needs a long time to cure them completely.

(3) Bronchitis and asthma : Use the relative apparatus of this invention to irradiate the parts of chest, back, windpipe and so on and the optimum treating dosage is selected on the basis of feeling comfortable for the patients. The conventional treating voltage is 100 - 180 volts. The distance between the generator of frequency spectrum and the affected parts is about 150 - 300 mm, and the curative effects may be achieved when the patient receives the treatment for 3 - 5 times and moreover, the symptoms will disappear for some patients but most of the patients will fully recover from these diseases after curing them for 15 - 30 days.

(4) Diseases in the department of gynaecology and obstetrics : To treat the disease of dysmenorrhoea is to use the relative apparatus of this invention to irradiate the abdomen or relative acupoints. The optimum treating dosage is selected on the basis of feeling comfortable for the patients. Ordinary patients may generally get the effect of disappearance or obvious relief from the disease when they have received the treatment only 1 - 2 times. To treat the diseases of pelvitis, wound caused by the caesarean operation and laceration in the vulva and birth canal is to use this apparatus to irradiate the affected parts directly. In the above methods, the disdance between the generator of frequency spectrum and the affected parts is about 50 - 500 mm and the treating voltage is 100 - 200 V. If this invention and above methods are adopted, the healing periods of operational and lacerated wounds will usually shorten 20% above compared with conventional methods.

Partial clinical cases finished by using experimental apparatus of this invention as main means are showen on Table 1 .

The other embodiments of this invention for the double-function lamp as as follows :

Example 1. The unilateral and single-group treatment-lighting lamp

This treatment lamp consists of the pedestal (21), lamp rod (22), support (23) and the single-group treatment lamp-holder (24) or general lighting bulb. The bottom of the lamp rod which is of plastic hose type is fixed on the pedestal (21) and an electric control system is mounted in this pedestal. A unilateral and single-group support (23) in which its electric parts are connected with the said electric control system is fixed on the top of the said rod.

The single-group treatment lamp-holder is made up in such a way : the rod-type matrix (26) is selected in the radiator (25), the transducing layer (27) of semiconductor-type is metallized on the said matrix, and meanwhile,the imitating genetic layer of human body's frequency spectrum which selects those element materials mentioned above in this specification is coated on the said transducing layer by means of coating method according to the said proportions in Group I. The matrix is mounted under the protection network (29) and in the centre of the reflex cover (30), and a threaded connector with threads as same as those of the ordinary illumination bulbs is allocated on the top of the said matrix (26), and is electrically connected with the said transducing layer (21).

Here the unilateral and single-group support adopts a unilateral cover (32) of general lighting lamps and its holding parts,and a lamp base (31) which is applicable both for general lighting bulb or single-group treatment lamp-holder is mounted in the support.When this lamp is used for lighting, a general threaded bulb is connected with the said lamp base (31), but when it is used for curing, a single-group treatment lamp-holder (24) would take the place accordingly.

Owing to the fact that the treatment lamp-holder is selected in which the imitating genetic layer (28) of human body's frequency spectrum is made up the element materials mentioned in Group I, therefore the lamp can offer notable curative effects to cure diseases of the blood circulating system. If it is necessary to make this treatment-lighting lamp offer notable curative effects for curing of surgical hurts and skins as well as the arthritis, it should adopt the treatment lamp-holder making up by the element materials in Group II for the said genetic layer (28). And if it is necessary to make this treatment-lighting lamp offer notable curative effects for curing of the automatic neurosis as well,it should adopt a treatment lamp-holder making up by the element materials in group III for the said genetic layer (28) (similarly hereinafter ).

Example 2 The bilateral and single-group treatment-lighting lamp

Here the parts and coupling forms are the same as said in Example 1 except those in followings :

1. Two bilateral lamp covers (33) of general bulbs,which are mutually reversal back by back, and their fixed parts are selected for the support,i.e., the bilateral support (33);

2. A treatment lamp-holder in which the imitating genetic layer (28) is made from any of the said three groups of element material proportions is mounted on the said bilateral support, and moreover an illumination bulb (35) may be also mounted as well.

Example 3 A trihedral and two-group treatment-lighting lamp

Here, the bottom of a spring moving lamp rod (22) is connected with a long-type pedestal (21), and the top of said lamp rod (22) is connected with a trihedral and two-group support (23) of rolling-type. An electric control system is mounted in the tong-type pedestal (21) and it is connected with the electric parts of the said support of rolling-type.

The said support (23) of rolling-type is made by connecting a rolling support buckle (34) of gripping-type with three pieces of reflex covers (30) which show in gripping form. The said three reflex covers (30) are in circular arc themselves and connected in the form of that the transverse section is comprised by the convex parts of the reflex covers showing trihedral sides. Two radiators (25) are fixed on two of the said three reflex covers, and a lamp tube (35) is fixed on another. The imitating genetic layers (28) of the said two radiators (25) may be respectively made from any two of the said three groups of element materials and proportions.

Example 4 The tetrahedral and three-group treatment-lighting lamp

Here, the parts and coupling forms are the same as said in Example 3 except those in followings:

The rolling-type support (23) is made by connecting a rolling support buckle (34) of gripping-type with four reflex covers (30) in the gripping form. The said four reflex covers (30) are in circular arc themselves and connected in the form of that the transverse section is comprised by the convex parts of the reflex covers showing tetragonal form. The three radiators (25) with their imitating genetic layers (28) being repectively made from the said three groups of element materials and proportions are respectively fixed on three reflex covers of the said four covers while the other is fixed with an illumination tube as well.

Besides the above embodiments from this invention, the suitable range of this apparatus may be enlarged as well. It may be considered to use it in the aspects of more chronic and stubborn diseases or stimulate the natural frequency speatrum of animals and plants. On the other hand, the preparing forms and techniques may also be manifold, for instance, the surface of imitating may be sprayed with some protective films.

TABLE 1. THE PARTIAL CLINICAL CASES OF ILLNESSES TREATED

BY THIS MATCHING EFFECT-FIELD TREATMENT APPARATUS OF HUMAN

BODY'S FREQUENCY SPECTRUM

| THE KINDS OF DISEASES | FULLY RECOVERED | RELIEVED | HAVE NO EFFECTS |
|---|---|---|---|
| Chilblains | more than twenty thousand | the curative effect is 98% | |
| Frostbite(mainly about 1 or 2 degree frostbite) | more than 400 | the curative effect is 95% | |
| Ulcer caused by vasculitis on the lower limbs | 1 | 2 | |
| Ulcer caused by varix on the lower limbs | 2 | 3 | |
| Laceration in the vulva and | 8 | 7 | |
| Healing of the wound caused by the caesarean operation | 6 | | |
| Burns or scalds | 176 | 41 | 3 |
| Phlegmon | 5 | 18 | |
| Anal fistula | 3 | 12 | |
| Bed sores | 12 | 41 | 2 |
| Herpes zonster | 54 | 15 | |
| Pneumonia of children | 11 | 2 | |
| Brochitis | 5 | 12 | |
| Bronchial asthma | 6 | 13 | |
| Primary hypertension | 4 | 7 | |
| Dysmenorrhoea | 19 | 57 | |

( to be continued )

| THE KINDS OF DISEASES | FULLY RECOVERED | RELIEVED | HAVE NO EFFECTS |
|---|---|---|---|
| Pelvitis | 3 | 21 | |
| Acute or chronic contusion of soft tissue | 247 | 89 | |
| Periarthritis of the shoulder | 11 | 43 | |
| Arthritis | 4 | 41 | 5 |
| Neurodermatitis | 12 | 35 | 2 |
| Rhinitis | 3 | 29 | |
| Relief of common cold | 4 | 22 | 3 |
| Headache | 27 | 21 | 4 |
| Stomachache | 20 | 14 | |
| Hepatitis type A | 1 | | |
| Hepatalgia or biliary pain | | 5 | |
| Chalazion | 8 | | |
| Chronic diarrhea | 10 | 27 | |
| Beriberi | 8 | 24 | |
| Hemiplegia | | 1 | |

## Claims

1. Method of producing an apparatus for radiation treatment matched with the human body's frequency spectrum which comprises forming a heat-resisting insulation on a matrix (13) of a radiation generator and control circuits,

   wherein

   a. a transducing layer (12) electrically connected to said circuits for transforming electrical energy into heat is set up on the said matrix (13) of the radiation generator, and then an imitating layer (11) for imitating the human body's frequency spectrum is set up on the transducing layer (12),

   b. the said transducing layer (12) is made-up of selected chlorides in a high-temperature solution method,

   c. the said imitating layer (11) is made-up of magnesium oxide, iron oxide, molybdenum oxide, zinc oxide, copper oxide, chromium oxide, metal-chromium, and, optionally, manganese oxide, lithium oxide, titanium oxide, strontia, cobalt oxide, vanadium oxide, and mixed rare-earth materials such as lanthana, by means of pulverization-calcination-smearing,

   d. the effective installation size of said transducing layer (12) on the said matrix (13) is larger than or equal to the transducing area of transducing layer (12), the transducing area of the transducing layer is larger than or equal to the area of the imitating layer (11), and the area of the imitating genetic layer (11) of human body's frequency spectrum is larger than or equal to the area of the effected part of the patient.

2. Method as in claim 1, **characterized** in that in said high-temperature solution method tin tetrachloride, stibium trichloride and iron trichloride are used in the proportions (80 - 120) : (0.5 - 2) : (0.3 - 2.5), and a preferred proportion is tetrachloride : stibium trichloride : iron trichloride = 105 : 0.8 : 1.

3. Method as in claim 1 or 2, **characterized** in that in said high temperature solution method the said chlorides are sufficiently mixed in determined proportions, then homogeneously sprayed on the clean matrix (13) and thereafter heated on temperatures of between 600 - 900 °C, preferably between 750 - 800 °C for 1 - 6 hours, preferably for 3 hours.

4. Method as in one of claims 1 to 3, **characterized** in that the materials forming said imitating layer are used in proportions as follows: magnesium oxide : iron oxide : manganese oxide : molybdenum oxide : zinc oxide : lithium oxide : copper oxide : titanium oxide : strontia : chromium oxide : cobalt oxide : vanadium oxide : chromium : mixed rare-earth materials such as lanthana = (0.5 - 8) : (7 - 30) : (0 - 6) : (0.6 - 5) : (1 - 17) : (0 - 4) : (1 - 7) : (0 - 7) : (0 - 5.5) : (25 - 85) : (0 - 5) : (0 - 10) : (0.5 - 4) : (0 - 40), the preferred proportions being selected according to different curative effects required to cure certain sorts of diseases.

5. Method as in claim 4, **characterized** in that the preferred proportions are selected as follows:
   a. to cure various diseases: 1 : 20 : 2 : 3 : 4 : 0.5 : 1 : 1.5 : 0.5 : 45 : 0.5 : 10 : 1 : 10,
   b. to cure the diseases of the human blood circulation system:
   2 : 30 : 0 : 0.6 : 6 : 1.5 : 1.2 : 2 : 1.2 : 37 : 0.3 : 5 : 1.2 : 12,
   c. to heal wounds and cure the diseases of skin, injury of soft tissue, arthritis and so on:
   0.5 : 25 : 3 : 2 : 7 : 2 : 1.5 : 1 : 1 : 55 : 0 : 0 : 1.5 : 0.5,
   d. to cure the diseases of autonomic neurosis:
   1.5: 15 : 1 : 1.5 : 5 : 0 : 1.6 : 0 : 0 : 62 : 0.7 : 3 : 0.7 : 8.

6. Method as in one of the preceding claims, **characterized** in that in said pulverization-calcination-smearing the selected materials are divided into groups or combined into one group and homogenously fixed, the materials pulverized into mixed particles with grain sizes of 40 - 80, preferably 45 - 50 mesh by using a ball grinder, then the materials are calcined for 2 - 6 hours, preferably for 3 hours at high temperature of 1100 - 1300 °C, preferably at 1100 - 1140 °C, the calcined materials are again pulverized into homogeneously mixed particles with a grain size of 40 - 80, preferably 45 - 50 mesh, thereafter a binder such as silicasol is put into the homogeneously mixed particles and mixed with these particles by homogeneous stirring, and finally, the mixture of particles and said binder is smeared onto the said clean transducing layer (12) by way of being stratified into some sublayers or merged into only one stratification and being divided into some groups or merged into one group by means of vacuum plating or plasma spraying or other methods of smearing.

7. Apparatus for radiation treatment matched with the human body's frequency spectrum which comprises a matrix (13) of a radiation generator made up of heat-resisting and insulating materials, control circuits, mechanical supports and a protective system of heating parts, wherein
   a. a transducing layer (12) of a high temperature chloride solution electrically connected to said circuits for transforming electrical energy into heat is set up on the said matrix (13), and then an imitating layer (11) for imitating the human body's frequency spectrum is set up on the transducing layer (12),
   b. the imitating layer (11) is formed by a thick-film layer which is made up from magnesium oxide, iron oxide, molybdenum oxide, zinc oxide, copper oxide, chromium oxide, metal-chromium and, optionally, manganese oxide, lithium oxide, titanium oxide, strontia, cobalt oxide, vanadium oxide, and mixed rare-earth materials such as lanthana,
   c. a stereophonic playback system and musical current acupoint-stimulating instrument as well as the control circuits are installed in the apparatus,
   d. mechanical parts for carrying the effect-field generator (18) are connected fixedly or movably with a frame of the apparatus.

8. Apparatus as in claim 7, **characterized** in that said mechanical parts comprise a steric console model or an orbital sliding-bed model or a fixed-wall model, respectively, according to the requirements.

**9.** Double-function treatment lamp, comprising a pedestal (21), a lamp rod (22), a lamp cover (32), a bulb or tube (35) and a control system of power supply, wherein

a. a mechanical support (23) of the treatment lamp is connected with the top of the said lamp rod (22),

b. one to three rod-type or strip-type radiators (25) of the treatment lamp are fixed on the fixed parts of the said support (23), and the said radiators are superimposed sequentially by a matrix (26), a transducing layer (27) electrically connected to the control system for transforming electrical energy into heat and an imitating layer (28) for imitating the human body's frequency spectrum or are made by covering the imitating layer (28) on a heat-resistant insulating tube in which heating resistance wires are fixed and wherein, the said imitating layer (28) is made of magnesia, iron oxide, molybdena, zinc oxide, copper oxide, oxide of chromium, metal-chromium and, optionally, black manganese, lithia, oxide of titanium, strontia, oxide of cobalt, oxide of vanadium, and mixed rare-earth materials such as lanthana.

**10.** Lamp as in claim 9, **characterized** in that the said mechanical support of the treatment lamp is selected according to the requirements under following constructions:

a. a unilateral and single-group support, i.e., a support (22) with only one lamp base (31) in which a treatment lampholder or a lighting bulb can be mounted, the support being connected with the angle-neck of a conical reflex cover (30) at the centre of the coning angle of the reflex cover (Fig. 3),

b. a bilaterial and single-group support (23), i. e., a support in which the radiator lamp base (31) for mounting a treatment lampholder is fixed with the lamp-base in different directions or opposite directions back by back, the said radiator lamp base being connected with the angle-neck end of the conical reflex cover (30) at the centre of the coning angle of the said reflex cover (30) (Fig. 5),

c. a trihedral and two-group support (23) of rolling - type, i. e., a support in which two said matrixes (26) and one support (23) of lighting lamp are fixed on a rolling support buckle (34) according to their mutual position relationship with their transverse sections forming a trilateral figure (Fig. 7),

d. a tetrahedral and three-group support (23) of rolling-type, i. e., a support in which three said matrixes (26) and one support (23) of lighting lamp are fixed on a rolling support buckle (34) according to their mutual position relationship with their transverse sections making up a tetragonal figure (Fig. 8).

**11.** Lamp as in claim 10, **characterized** in that an edge of said rolling support buckle (34) is fixed on the top of the said lamp rod (22) by means of rolling clip parts and stays.

**12.** Apparatus or lamp as in one of claims 7 to 11, **characterized** in that said imitating layer (28) is made of materials selectively in three groups of proportions thereof as specified in features b., c. or d., respectively, of claim 5.

**Patentansprüche**

**1.** Verfahren zum Herstellen eines Gerätes für die Behandlung mit einer Strahlung, welche an das Frequenzspektrum des menschlichen Körpers angepaßt ist, welches das Bilden einer wärmebeständigen Isolierung auf einer Matrix (13) eines Strahlungsgenerators und von Steuerschaltungen umfaßt, wobei

a) eine Wandlerschicht (12), welche elektrisch mit den Schaltungen zum Umwandeln von elektrischer Energie in Wärme verbunden ist, auf der Matrix (13) des Strahlungsgenerators aufgebaut wird und dann eine Imitierschicht (11) zum Imitieren des Frequenzspektrums des menschlichen Körpers auf der Wandlerschicht (12) aufgebaut wird,

b) die Wandlerschicht (12) aus ausgewählten Chloriden in einem Hochtemperatur-Lösungsverfahren gebildet wird,

c) die Imitierschicht (11) aus Magnesiumoxid, Eisenoxid, Molybdenoxid, Zinkoxid, Kupferoxid, Chromoxid, metallischem Chrom und fakultativ Manganoxid, Lithiumoxid, Titanoxid, Strontiumoxid, Kobaltoxid, Vanadiumoxid und gemischten Seltenerd-Materialien, wie Lanthanoxid, mit Hilfe von Pulverisieren-Brennen-Aufstreichen gebildet wird,

d) die effektive Installationsgröße der Wandlerschicht (12) auf der Matrix (13) größer oder gleich der Wandlerfläche der Wandlerschicht (12) ist, wobei die Wandlerfläche der Wandlerschicht größer oder gleich der Fläche der Imitierschicht (11) ist und die Fläche der genetischen Imitierschicht (11) für das Frequenzspektrum des menschlichen Körpers größer oder gleich der Fläche des beeinträchtigten Körperteils des Patienten ist.

2. Verfahren nach Anspruch 1, dadurch gekennzeichnet, daß bei dem Hochtemperatur-Lösungsverfahren Zinntetrachlorid, Antimontrichlorid und Eisentrichlorid im Verhältnis (80 - 120): (0,5 - 2): (0, 3 - 2,5) verwendet werden und ein bevorzugtes Verhältnis Tetrachlorid : Antimontrichlorid : Eisentrichlorid = 105 : 0,8 : 1 ist.

3. Verfahren nach Anspruch 1 oder 2, dadurch gekennzeichnet, daß bei dem Hochtemperatur-Lösungsverfahren die Chloride in vorbestimmten Verhältnissen ausreichend gemischt werden, dann homogen auf die saubere Matrix (13) gesprüht werden und danach auf Temperaturen zwischen 600°C und 900°C, vorzugsweise zwischen 750°C und 800°C, für 1h bis 6h, vorzugsweise über 3h, erwärmt werden.

4. Verfahren nach einem der Ansprüche 1 bis 3, dadurch gekennzeichnet, daß die Materialien, welche die Imitierschicht bilden, in den folgende Verhältnissen verwendet werden:
Magnesiumoxid : Eisenoxid : Manganoxid : Molybdenoxid : Zinkoxid : Lithiumoxid : Kupferoxid : Titanoxid : Strontiumoxid : Chromoxid : Kobaltoxid : Vanadiumoxid : Chrom : gemischte Seltenerd-Materialien wie Lanthanoxid = (0,5 - 8) : (7 - 30) : (0 - 6) : (0,6 - 5) : (1 - 17) : (0 - 4) : (1 - 7) : (0 - 7) :
(0 - 5,5) : (25 - 85) :
(0 - 5): (0 - 10) : (0,5 - 4) : (0 - 40);
wobei die bevorzugten Verhältnisse entsprechend den verschiedenen Heileffekten gewählt werden, die nötig sind, um bestimmte Arten von Krankheiten zu heilen.

5. Verfahren nach Anspruch 4, dadurch gekennzeichnet, daß die bevorzugten Verhältnisse wie folgt gewählt werden:
a) zum Heilen verschiedener Krankheiten:
1 : 20: 2: 3: 4: 0,5: 1: 1,5: 0,5: 45: 0,5: 10: 1: 10,
b) zum Heilen von Krankheiten des menschlichen Blutkreislaufsystems:
2: 30: 0: 0,6: 6: 1,5: 1,2: 2: 1,2: 37: 0,3: 5: 1,2: 12,
c) zum Heilen von Wunden und zum Heilen von Krankheiten der Haut, Verletzungen des weichen Gewebes, Arthritis usw.:
0,5: 25: 3: 2: 7: 2: 1,5: 1: 1: 55: 0: 0: 1,5: 0,5,
d) zum Heilen der Krankheiten der autonomen Neurose:
1,5: 15: 1: 1,5: 5: 0: 1,6: 0: 0: 62: 0,7: 3: 0,7: 8.

6. Verfahren nach einem der vorangehenden Ansprüche, dadurch gekennzeichnet, daß bei dem Pulverisieren-Brennen-Aufstreichen die ausgewählten Materialien in Gruppen aufgeteilt werden oder in einer Gruppe zusammengefaßt werden und homogen fixiert werden, die Materialien zu gemischten Teilchen mit Korngrößen von 40 - 80 Mesh, vorzugsweise mit 45 - 50 Mesh mit Hilfe einer Kugelmühle, pulverisiert werden, die Materialien dann für 2 h - 6 h, vorzugsweise über 3 h, bei einer hohen Temperatur von 1.100°C - 1.300°C, vorzugsweise bei 1.100°C - 1.140°C, gebrannt werden, die gebrannten Materialien wieder zu homogen gemischten Teilchen mit einer Korngröße von 40 - 80 Mesh, vorzugsweise 45 - 50 Mesh, pulverisiert werden, danach ein Bindemittel, wie Kieselsol, zu den homogen gemischten Teilchen zugefügt wird und mit diesen Teilchen durch homogenes Rühren vermischt wird und schließlich die Mischung der Teilchen und des Bindemittels auf die saubere Wandlerschicht (12) aufgestrichen wird, indem sie in einigen Unterschichten geschichtet wird oder in einer einzigen Schichtung vereinigt wird und mit Hilfe von Vakuumbeschichtung oder Plasmasprühen oder anderen Methoden des Aufstreichens in einige Gruppen aufgeteilt oder in einer Gruppe vereinigt wird.

7. Vorrichtung für die Behandlung mit einer Strahlung, welche an das Frequenzspektrum des menschlichen Körpers angepaßt ist, welche eine Matrix (13) eines Strahlungsgenerators, die aus wärmebeständigen isolierenden Materialien besteht, Steuerschaltungen, mechanische Trägerelemente und ein Schutzsystem für die heizenden Teile umfaßt, wobei
a) eine Wandlerschicht (12) aus einer Hochtemperatur-Chloridlösung, welche elektrisch mit den Schaltungen zum Umwandeln von elektrischer Energie in Wärme verbunden ist, auf der Matrix (13) angebracht ist und dann eine Imitierschicht (11) zum Imitieren des Frequenzspektrums des menschlichen Körpers auf der Wandlerschicht (12) angebracht ist,

b) die Imitierschicht (11) durch eine Dickfilmschicht gebildet wird, welche aus Magnesiumoxid, Eisenoxid, Molybdenoxid, Zinkoxid, Kupferoxid, Chromoxid, metallischem Chrom und fakultativ Manganoxid, Lithiumoxid, Titanoxid, Strontiumoxid, Kobaltoxid, Vanadiumoxid und gemischten Seltenerd-Materialien wie Lanthanoxid besteht,

c) ein Stereophonie-Wiedergabesystem und ein Akupunkte anregendes Musikabgabegerät ebenso wie die Steuerschaltungen in dem Gerät installiert sind,

d) mechanische Teile zum Tragen des Generators des effektiven Felds (18) fest oder beweglich mit einem Rahmen des Geräts verbunden sind.

8. Vorrichtung nach Anspruch 7, dadurch gekennzeichnet, daß die mechanischen Teile ein sterisches Konsolenmodell oder ein Orbital-Gleitbett-Modell oder ein Festwand-Modell jeweils entsprechend den Anforderungen umfassen.

9. Doppelfunktions-Behandlungslampe, welche einen Sockel (21), eine Lampenstange (22), eine Lampenabdeckung (32), eine Birne oder Röhre (35) und ein Steuersystem für die Leistungsversorgung umfaßt, wobei

a) ein mechanischer Träger (23) der Behandlungslampe mit dem Oberteil der Lampenstange (22) verbunden ist,

b) ein bis drei stabartige oder streifenartige Radiatoren (25) der Behandlungslampe an den festen Teilen des Trägers (23) befestigt sind und diese Radiatoren sequentiell durch eine Matrix (26), eine Wandlerschicht (27), welche elektrisch mit dem Steuersystem zum Umwandeln von elektrischer Energie in Wärme verbunden ist, und eine Imitierschicht (28) zum Imitieren des Frequenzspektrums des menschlichen Körpers geschichtet sind oder durch Bedecken der Imitierschicht (28) auf einer wärmebeständigen isolierenden Röhre, in welcher Heizwiderstandsdrähte befestigt sind, hergestellt sind, wobei die Imitierschicht (28) aus Magnesiumoxid, Eisenoxid, Molybdenoxid, Zinkoxid, Kupferoxid, Chromoxid, metallischem Chrom und fakultativ Manganschwarz, Lithiumoxid, Titanoxid, Strontiumoxid, Kobaltoxid, Vanadiumoxid und gemischten Seltenerd-Materialien wie Lanthanoxid besteht.

10. Lampe nach Anspruch 9, dadurch gekennzeichnet, daß der mechanische Träger der Behandlungslampe entsprechend den Erfordernissen aus den folgenden Konstruktionen ausgewählt ist:

a) einem unilateralen Einzelgruppen-Träger, d.h. einem Träger (22) mit nur einer Lampenbasis (31), in welcher ein Behandlungslampenhalter oder eine Beleuchtungsbirne montiert werden kann, wobei der Träger mit dem Winkelhals einer konischen Reflektierabdeckung (30) im Zentrum des Konuswinkels der reflektierenden Abdeckung verbunden ist (Fig. 3),

b) einem zweiseitigen Einzelgruppen-Träger (23), d.h. einem Träger, in welchem die Radiatorlampenbasis (31) zum Montieren eines Behandlungs-Lampenhalters in einer anderen Richtung als der der Lampenbasis oder in der entgegengesetzten Richtung, Rücken an Rücken, angebracht ist, wobei die Radiatorlampenbasis mit dem Winkelhals-Ende der konischen reflektierenden Abdeckung (30) im Zentrum des konischen Winkels der reflektierenden Abdeckung (30) (Fig. 5) verbunden ist,

c) einem dreiflächigen Zwei-Gruppen-Träger (23) eines drehbaren Typs, d.h. einem Träger, in welchem die zwei Matrizen (26) und ein Träger (23) einer Beleuchtungslampe an einem drehbaren Trägerschild (34) entsprechend ihrer gegenseitigen Positionsbeziehung befestigt sind, wobei ihre transversalen Abschnitte eine dreiflächige Form bilden (Fig. 7),

d) einem vierflächigen Drei-Gruppen-Träger (23) eines drehbaren Typs, d.h. einem Träger, in welchem drei Matrizen (26) und ein Träger (23) einer Beleuchtungslampe an einem drehbaren Trägerschild (34) entsprechend ihrer gegenseitigen Positionsbeziehung befestigt sind, wobei ihre transversalen Abschnitte eine vierflächige Figur bilden (Figur 8).

11. Lampe nach Anspruch 10, dadurch gekennzeichnet, daß eine Seite des drehbaren Trägerschilds (34) an der Oberseite der Lampenstange (22) mit Hilfe von drehbaren Halteteilen und Bolzen befestigt ist.

12. Vorrichtung oder Lampe nach einem der Ansprüche 7 bis 11, dadurch gekennzeichnet, daß die Imitierschicht (28) aus Materialien selektiv in drei Gruppen von Verhältnissen hergestellt ist, wie sie in den Merkmalen b), c) bzw. d) von Anspruch 5 angegeben sind.

## Revendications

1. Un procédé de fabrication d'un appareil de traitement par rayonnements accordés au spectre de fréquences du corps humain qui comprend la formation d'une couche isolante résistant à la chaleur sur une matrice (13) d'un générateur de rayonnements et de circuits de commande dans lequel:

   a. une couche transductrice, connectée électriquement auxdits circuits pour transformer l'énergie électrique en chaleur est placée sur ladite matrice (13) du générateur de rayonnements, puis une couche (11) générant ou reproduisant le spectre de fréquences du corps humain est placée sur la couche transductrice (12);

   b. ladite couche transductrice (12) est préparée à partir de chlorures sélectionnés préparés en solution à haute température;

   c. ladite couche générant le spectre de fréquences (11) est constituée d'oxyde de magnésium, d'oxyde de fer, d'oxyde de molybdène, d'oxyde de zinc, d'oxyde de cuivre, d'oxyde de chrome, de chrome métallique, éventuellement d'oxyde de manganèse, d'oxyde de lithium, d'oxyde de titane, d'oxyde de strontium, d'oxyde de cobalt, d'oxyde de vanadium et de matières à base de mélanges de terres rares, tels que des oxydes de lanthane, par étalementpulvérisation-calcination;

   d. la dimension efficace de ladite couche transductrice (12) sur ladite matrice (13) est supérieure ou égale à la surface transductrice de la couche transductrice (12), la surface transductrice de la couche transductrice est supérieure ou égale à la surface de la couche générant le spectre de fréquences (11) et la surface de la couche générant le spectre de fréquences (11) du corps humain est supérieure ou égale à la surface des parties affectées dudit patient.

2. Procédé selon la revendication 1, caractérisé en ce que dans ladite étape de mise en solution à haute température, on utilise le tétrachlorure d'étain, le trichlorure d'antimoine et le trichlorure de fer dans des proportions de 80-120/0,5-2/0,3-2,5, les proportions préférées étant tétrachlorure d'étain/trichlorure d'antimoine/trichlorure de fer 105/0,8/1.

3. Procédé selon la revendication 2, caractérisé en ce que dans cette étape de mise en solution à haute température, lesdits chlorures sont mélangés suffisamment dans des proportions déterminées puis pulvérisés de façon homogène sur la matrice nettoyée (13) et ensuite chauffés à une température de 600 à 900°C, de préférence de 750°C à 800°C pendant 1 à 6 heures, de préférence pendant 3 heures.

4. Procédé selon l'une des revendications 1 à 3, caractérisé en ce que les matériaux formant ladite couche générant les spectres de fréquence sont utilisés dans les proportions respectives suivantes: oxyde de magnésium/oxyde de fer/oxyde de manganèse/oxyde de molybdène/oxyde de zinc/oxyde de lithium/oxyde de cuivre/oxyde de titane/oxyde de strontium/oxyde de chrome/oxyde de cobalt/oxyde de vanadium/chrome/matériaux des mélanges de terres rares, tels que des oxydes de lanthane: 0,5-8/7-30/0-6/0,6-5/1-17/1-4/1-7/0-7/0-5,5/25-85/0-5/0-10/0,5-4/0-40, les proportions préférées étant sélectionnées en fonction des différents effets curatifs nécessaires pour soigner les différents types de maladies.

5. Procédé selon la revendication 4, caractérisé en ce que les proportions préférées sont choisies comme suit:

   a. pour soigner les différentes maladies:
   1/20/2/3/4/0,5/1/1,5/0,5/45/0,5/10/1/10;

   b. pour soigner les maladies associées au système de circulation sanguine de l'homme:
   2/30/0/0,6/6/1,5/1,2/2/1,2/37/0,3/5/1,2/12;

   c. pour soigner les plaies et traiter les maladies de peau, les lésions des tissus mous, l'arthrite, etc:
   0,5/25/3/2/7/2/1,5/1/1/55/0/0/1,5/0,5;

   d. pour traiter la névrose autonome:
   1,5/15/1/1,5/5/0/1,6/0/0/62/0,7/3/0,7/8.

6. Procédé selon l'une quelconque des revendications précédentes, caractérisé en ce que dans ledit procédé d'étalement-pulvérisation-calcination, les matériaux sélectionnés sont divisés en groupes ou combinés en un seul groupe et liés de façon homogène, les matériaux sont pulvérisés pour former des mélanges de particules dont la dimension des grains est de 40 à 80 mesh, de préférence 45 à 50 mesh (mailles de 0,322 à 0,287mm) par utilisation d'un broyeur à billes, puis les matériaux sont calcinés pendant 2 à 6 heures, de préférence pendant 3 heures, à une température élevée de 1100 à 1300°C, de préférence à une température de 1100 à 1140°C, les matériaux calcinés étant alors à nouveau pulvérisés pour former un mélange de particules homogènes présentant une dimension de grains de 40 à 80 mesh et de préférence de 45 à 50 mesh (mailles de 0,322 à 0,287mm), puis un liant tel qu'un sel de silice est ajouté au mélange de particules homogènes et mélangé à ces particules homogénes sous agitation et finalement le mélange des particules et dudit liant est étalé sur ladite couche transductrice nettoyée (12) par stratification en plusieurs sous-couches ou fusionné en une seule sous-couche et est divisé en plusieurs groupes ou fusionnée en un seul groupe par revêtement sous vide ou pulvérisation par plasma ou d'autres méthodes d'étalement.

7.  Appareil de traitement par rayonnements accordés au spectre de fréquences du corps humain qui comprend une matrice (13) d'un générateur de rayonnements constituée de matériaux isolants et résistants à la chaleur, de circuits de commande, de supports mécaniques et d'un système de protection des parties chauffantes, dans lequel:

a. une couche transductrice (12) de solution de chlorure à haute température, connectée électriquement audit circuit pour transformer l'énergie électrique en chaleur est placée sur ladite matrice (13), puis une couche (11) générant le spectre de fréquences du corps humain est placée sur la couche transductrice (12);

b. la couche générant le spectre de fréquences du corps humain (11) est formée d'une couche ou film épais qui est constituée d'oxyde de magnésium, d'oxyde de fer, d'oxyde de molybdène, d'oxyde de zinc, d'oxyde de cuivre, d'oxyde de chrome, de chrome métallique, éventuellement d'oxyde de manganèse, d'oxyde de lithium, d'oxyde de titane, d'oxyde de strontium, d'oxyde de cobalt, d'oxyde de vanadium et de matières à base de mélanges de terres rares, tels que des oxydes de lanthane;

c. un système stéréophonique de pré-sonorisation et un instrument musical de stimulation instantanée des points d'acupuncture ainsi que des circuits de commande sont installés dans l'appareil;

d. les parties mécaniques pour réaliser le générateur à effet de champs (18) sont connectées de façon fixe ou mobile à un cadre de l'appareil.

8.  Appareil selon la revendication 7, caractérisé en ce que lesdites parties mécaniques sont respectivement du type à console stérique ou un modèle orbital coulissant ou un modèle à paroi fixe, respectivement selon les exigences.

9.  Lampe de traitement à double fonction comprenant une embase (21), une tige de lampe (22), un chapeau de lampe (32), un tube ou une ampoule (35) et un dispositif de commande de l'alimentation électrique dans laquelle:

a. un support mécanique (23) de la lampe de traitement est connecté avec le sommet de ladite tige de lampe (22);

b. un des trois éléments émettant les rayonnements, de type barre ou bande (25, de la lampe de traitement est fixé sur les parties fixes dudit support (23) et lesdits éléments émettant les rayonnements sont recouverts séquentiellement par une matrice (26), une couche transductrice (27) électriquement connectée au système de commande de façon à transformer l'énergie électrique en chaleur, et une couche générant le spectre de fréquences du corps humain (28) ou bien sont préparés par dépôt de la couche générant le spectre de fréquences (28) sur un tube isolant résistant à la chaleur dans lequel les fils de résistance chauffant sont fixés et dans lequel ladite couche générant le spectre de fréquences (28) est constituée de magnésie, oxyde de fer, molybdène, oxyde de zinc, oxyde de cuivre, oxyde de chrome, chrome métallique et éventuellement manganèse noir, oxyde de lithium, oxyde de titane, oxyde de strontium, oxyde de cobalt, oxyde de vanadium et des matières à base de mélanges de terres rares, tels que oxydes de lanthane.

10. Lampe selon la revendication 9, caractérisée en ce que ledit support mécanique de la lampe de traitement est sélectionné selon les exigences parmi les structures suivantes:

a. un support à groupe unique et unilatéral, c'est-à-dire un support (22) comportant seulement une embase de lampe (31) dans lequel un support de lampe de traitement ou une ampoule d'éclairage peuvent être montés, le support étant connecté avec le col d'angle du chapeau réfléchissant conique (30) au centre de l'angle conique du chapeau réfléchissant (figure 3);

b. un support bilatéral à groupe unique (23), c'est-à-dire un support dans lequel l'embase (31) des lampes émettant les rayonnements, pour réaliser un support de lampe de traitement, est fixé dos à

16

dos sur l'embase de la lampe selon différentes directions ou dans des directions opposées, ladite embase de la lampe à rayonnements étant connectée à l'extrémité du col d'angle du chapeau réfléchissant conique (30) au centre de l'angle conique dudit chapeau réfléchissant (30) (figure 5);

c. un support à deux groupes et à trois faces (23) du type tournant, c'est-à-dire un support dans lequel les deux matrices (26) et un support (23) de la lampe d'éclairage sont fixés sur un étrier de support tournant (34) en fonction de leur localisation mutuelle, leurs sections transversales formant une structure à trois faces (figure 7);

d. un support à trois groupes et à quatre faces (25) du type tournant, c'est-à-dire un support sur lequel les trois matrices (26) et un support (23) de la lampe d'éclairage sont fixés sur un étrier de support tournant (34) en fonction de leur localisation mutuelle, leurs sections transversales formant une structure à quatre faces.

**11.** Lampe selon la revendication 10, caractérisée en ce qu'un bord dudit étrier tournant (34) est fixé au sommet de ladite tige de lampe (22) par des parties et des supports de pinces tournantes.

**12.** Un appareil ou une lampe selon l'une quelconque des revendications 7 à 11, caractérisé en ce que ladite couche générant le spectre (28) est constituée de matériaux sélectionnés dans trois groupes dont les proportions sont spécifiées dans les points b, c ou d, respectivement, de la revendication 5.

Fig.1

Fig.2

Fig.3

$A-A$
Enlarged

Fig.4

Direction A

A

35

31

33

24

23

Fig.5

23

34

22

21

Fig.6

Fig.7

I—A

23  34

I—A

$$\frac{A-A}{2:1}$$

25

25

30

35

Fig.8